(19) Europäisches Patentamt
European Patent Office
Office européen des brevets

(11) EP 1 581 227 B1

(12) **EUROPEAN PATENT SPECIFICATION**

(45) Date of publication and mention of the grant of the patent:
**28.02.2007 Bulletin 2007/09**

(21) Application number: **04700048.4**

(22) Date of filing: **02.01.2004**

(51) Int Cl.:
***A61K 31/50*** *(2006.01)* ***A61P 13/12*** *(2006.01)*

(86) International application number:
**PCT/FI2004/000002**

(87) International publication number:
**WO 2004/060375 (22.07.2004 Gazette 2004/30)**

# (54) A METHOD FOR TREATING RENAL FAILURE

METHODE ZUR BEHANDLUNG VON NIERENINSUFFIZIENZ

PROCEDE POUR TRAITER L'INSUFFISANCE RENALE

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **03.01.2003 FI 20030015**

(43) Date of publication of application:
**05.10.2005 Bulletin 2005/40**

(73) Proprietor: **Orion Corporation**
**02200 Espoo (FI)**

(72) Inventors:
- **KIVIKKO, Matti**
**FIN-00200 Helsinki (FI)**
- **HAIKALA, Heimo**
**FIN-02180 Espoo (FI)**

(74) Representative: **Sexton, Jane Helen**
**J.A. KEMP & CO.**
**14 South Square**
**Gray's Inn**
**London WC1R 5JJ (GB)**

(56) References cited:
**EP-A- 0 565 546**

- **DATABASE WPI Section Ch, Week 199314 Derwent Publications Ltd., London, GB; Class B03, AN 1993-111711 XP002283338 & JP 04 368328 A (TEIKOKU HORMONE MFG CO LTD) 21 December 1992 (1992-12-21)**
- **PAGEL P S ET AL: "PHARMACOLOGY OF LEVOSIMENDAN: A NEW MYOFILAMENT CALCIUM SENSITIZER" CARDIOVASCULAR DRUG REVIEWS, NEVA PRESS, BRANFORD, CT, US, vol. 14, no. 3, 1996, pages 286-316, XP008030915 ISSN: 0897-5957**
- **PAGEL P S HETTRICK D A ET AL: "INFLUENCE OF LEVOSIMENDAN PIMOBENDAN, AND MILRINONE ON THE REGIONAL DISTRIBUTION OF CARDIAC OUTPUT IN ANAESTHETIZED DOGS" 1996, BRITISH JOURNAL OF PHARMACOLOGY, BASINGSTOKE, HANTS, GB, PAGE(S) 609-615 , XP002901958 ISSN: 0007-1188 abstract page 611, column 1, paragraph 3**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 581 227 B1

## Description

Technical field

The present invention relates to the treatment of renal failure by administering levosimendan or its metabolite (II) or any of their pharmaceutically acceptable salts, to a mammal in need of such treatment.

Background of the invention

Levosimendan, which is the (-)-enantiomer of [[4-(1,4,5,6-tetrahydro-4-methyl-6-oxo-3-pyridazinyl)phenyl]hydrazono]propanedinitrile, and the method for its preparation is described in EP 565546 B1. Levosimendan is potent in the treatment of heart failure and has significant calcium dependent binding to troponin. Levosimendan is represented by the formula:

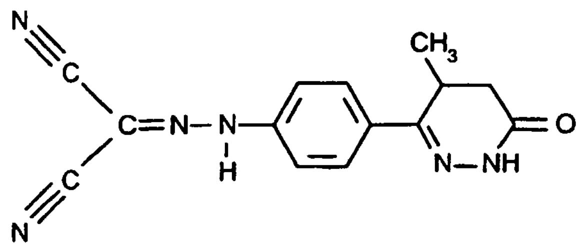

I

The hemodynamic effects of levosimendan in man are described in Sundberg, S. et al., Am. J. Cardiol., 1995; 75: 1061-1066 and in Lilleberg, J. et al., J. Cardiovasc. Pharmacol., 26(Suppl.1), S63-S69, 1995. Pharmacokinetics of levosimendan in man after i.v. and oral dosing is described in Sandell, E.-P. et al., J. Cardiovasc. Pharmacol., 26(Suppl. 1), S57-S62, 1995. Clinical studies have confirmed the beneficial effects of levosimendan in heart failure patients.

Recently it has been found that levosimendan has an active metabolite (R)-N-[4-(1,4,5,6-tetrahydro-4-methyl-6-oxo-3-pyridazinyl)phenyl]acetamide(II) which is present in human following administration of levosimendan. The effects of (II) are similar to levosimendan. The use of (II) for increasing calcium sensitivity of contractile proteins in the cardiac muscle has been described in WO 99/66932.

The mammalian renal system serves primary roles of both in the removal of catabolic waste products from the bloodstream and in the maintenance of fluid and electrolyte balances in the body. Renal failure is characterized by acute or chronic deterioration of kidney function. Renal failure is a life-threatening condition in which the build-up of catabolites and other toxins, and/or the development of significant imbalances in electrolytes or fluids, may lead to the failure of other major organ systems and death. Dialysis and kidney transplantation can be used as treatments, but these procedures can have serious complications.

Thus, there is a need for additional options to the presently available treatments for renal failure.

Summary of the invention

It has now been found that levosimendan or its active metabolite (II) or any of the pharmaceutically acceptable salts thereof are useful in the treatment of renal failure.

Therefore, the present invention provides the use of levosimendan or its active metabolite (II) or any of their pharmaceutically acceptable salts in the manufacture of a medicament for the treatment of renal failure.

The present invention also provides the use of levosimendan or its metabolite (II) or any of their pharmaceutically acceptable salts in the manufacture of a medicament for reducing mortality in a mammal suffering from renal failure the mortality being associated with the deterioration of kidney function.

Detailed description

The invention relates to the use of levosimendan for the manufacture of a medicament to reduce, inhibit or prevent symptoms of renal failure in a mammal, including man. The administration of levosimendan or its active metabolite (II) can be enteral, e.g. oral or rectal; parenteral, e.g. intravenous; or transdermal.

As used herein the term "renal failure" means a disease state or condition wherein the renal tissues fail to perform their normal functions. Renal failure includes chronic and acute renal failure or dysfunction.

Acute renal failure is broadly defined as a rapid deterioration in renal function sufficient to result in accumulation of nitrogenous wastes in the body. The causes of such deterioration include renal hypoperfusion, obstructive uropathy, and intrinsic renal disease such as acute glomerulonephritis.

Chronic renal failure is usually caused by renal injuries of a more sustained nature which often lead to progressive destruction of nephron mass. Glomerulonephritis, tubulointerstitial diseases, diapetic nephropathy and nephrosclerosis are among the most common causes of chronic renal failure. Chronic renal failure can be defined as a progressive, permanent and significant reduction in glomerular filtration rate (GFR) due to a significant and continuing loss ofnephrons. The clinical syndrome that results from profound loss of renal function is called uremia.

Diagnostic signs of renal failure include lower than normal creatinine clearance; lower than normal free water clearance; higher than normal blood urea and/or nitrogen and/or potassium and/or creatinine levels; altered activity of kidney enzymes such as gamma glutamyl synthetase; altered urine osmolarity or volume; elevated levels of microalbuminuria or macroalbuminuria; glomerular and arteriolar lesions; tubular dilation; hyperphosphatemia; or need for dialysis.

The inhibition of the renal failure can be evaluated by measuring these parameters in mammals by methods well known in the art, e.g. by measuring creatinine clearance.

Renal failure can be divided into several stages starting from mild form followed by moderate and severe forms and processing to so called end stage renal disease. These stages can be identified in a conventional way e.g. by determining the creatinine clearance values for which well-defined ranges are assigned to the different stages of renal insufficiency.

The effective amount of levosimendan or its active metabolite (II) to be administered to a subject depends upon the condition to be treated, the route of administration, age, weight and the condition of the patient. In general levosimendan is administered orally to man in daily dose from 0.1 to 20 mg, preferably from 0.2 to 15 mg, more preferably from 0.5 to 10 mg, given once a day or divided into several doses a day, depending on the age, body weight and condition of the patient. The oral daily dose of the active metabolite (II) in man is generally within the range of 0.05 - 10 mg.

Levosimendan can be administered by intravenous infusion using the infusion rate typically from 0.01 to 10 μg/kg/min, more typically from 0.02 to 5 μg/kg/min. For the intravenous treatment of renal failure an intravenous bolus of 10 - 200 μg/kg followed by infusion of 0.2 - 3 μg/kg/min may be needed. The active metabolite (II) can be administered intravenously using an infusion rate, which is from 0.001 to 1 μg/kg/min, preferably from 0.005 to 0.5 μg/kg/min.

Levosimendan or its active metabolite (II) is formulated into dosage forms suitable for the treatment of renal failure using the principles known in the art. It is given to a patient as such or preferably in combination with suitable pharmaceutical excipients in the form of tablets, dragees, capsules, suppositories, emulsions, suspensions or solutions whereby the contents of the active compound in the formulation is from about 0.5 to 100 % per weight. Choosing suitable ingredients for the composition is a routine for those of ordinary skill in the art. It is evident that suitable carriers, solvents, gel forming ingredients, dispersion forming ingredients, antioxidants, colours, sweeteners, wetting compounds, release controlling components and other ingredients normally used in this field of technology may be also used.

For oral administration in tablet form, suitable carriers and excipients include e.g. lactose, corn starch, magnesium stearate, calcium phosphate and talc. For oral administration in capsule form, useful carriers and excipients include e.g. lactose, corn starch, magnesium stearate and talc. For controlled release oral compositions release controlling components can be used. Typical release controlling components include hydrophilic gel forming polymers such as hydroxypropylmethyl cellulose, hydroxypropyl cellulose, carboxymethyl celluloses, alginic acid or a mixture thereof; vegetable fats and oils including vegetable solid oils such as hydrogenated soybean oil, hardened castor oil or castor seed oil (sold under trade name Cutina HR), cotton seed oil (sold under the trade names Sterotex or Lubritab) or a mixture thereof; fatty acid esters such as triglycerides of saturated fatty acids or their mixtures e.g. glyceryl tristearates, glyceryl tripalmitates, glyceryl trimyristates, glyceryl tribehenates (sold under the trade name Compritol) and glyceryl palmitostearic acid ester.

Tablets can be prepared by mixing the active ingredient with the carriers and excipients and compressing the powdery mixture into tablets. Capsules can be prepared by mixing the active ingredient with the carriers and excipients and placing the powdery mixture in capsules, e.g. hard gelatin capsules. Typically a tablet or a capsule comprises from 0.1 to 10 mg, more typically 0.2 to 5 mg, of levosimendan or its active metabolite (II).

Formulations suitable for intravenous administration such as injection or infusion formulation, comprise sterile isotonic solutions of levosimendan or its active metabolite (II) and vehicle, preferably aqueous solutions. Typically an intravenous infusion solution comprises from 0.01 to 0.1 mg/ml of levosimendan or its active metabolite (II).

The active ingredient of the invention may be administered periodically, e.g. weekly or biweekly, or daily or several times a day, depending on the patient's needs. The elimination half-life of the active metabolite (II) in man is rather long, about 72 h. Therefore, a periodical treatment, e.g. weekly, may be satisfactory.

Salts of levosimendan or its active metabolite (II) may be prepared by known methods. Pharmaceutically acceptable salts are useful as active medicaments, however, preferred salts are the salts with alkali or alkaline earth metals.

Examples

Pharmaceutical example.

Hard gelatin capsule size 3

| | |
|---|---|
| Levosimendan | 2.0 mg |
| Lactose | 198 mg |

The pharmaceutical preparation in the form of a capsule was prepared by mixing levosimendan with lactose and placing the powdery mixture in hard gelatin capsule.

Experiments

In total, 701 heart failure patients from levosimendan trials 3001021b and 3001030 were included into the analysis to determine the effect of levosimendan on mortality in patients with normal or impaired renal function.

The study 3001021b compared levosimendan with dobutamine in a double-blind, parallel-group, randomised trial in 203 patients with low-output heart. Treatment with levosimendan (n=103) was started with a loading dose of 24 μg/kg infused over 10 minutes and followed by a continuous infusion of 0.1 μg/kg/min. For patients in whom the cardiac index had not increased by at least 30% compared with the baseline value at 2 hours, the dose was doubled to 0.2 μg/kg/min for levosimendan (n=69). Infusions were continued for totally 24 hours. Mortality was followed prospectively up to 31 days and retrospectively up to 180 days.

The study 3001030 evaluated the safety of levosimendan in patients with left ventricular failure after an acute myocardial infarction. The study was a placebo-controlled, double-blind, parallel-group, randomised, trial in 504 patients. Levosimendan was administered as a 10-minute loading dose followed by a continuous infusion for a total of 6 hours. The following doses were used: 6 μg/kg + 0.1 μg/kg/min (n=103), 12 μg/kg + 0.2 μg/kg/min (n=100), 24 μg/kg + 0.2 μg/kg/min (n=99), 24 μg/kg + 0.4 μg/kg/min (n=100) and similar placebo infusion (n=102). All-cause mortality was followed prospectively up to 14-days and retrospectively up to 180-days.

The patients combined from the above studies were classified according to their baseline renal function. The formula of Cockroft and Gault (Lafayette et al, 1997) was used to calculate the creatinine clearance (CLcr) values from the baseline serum creatinine) values as follows:

$$\text{for men:} \quad CLcr = \frac{(140 - \text{age in years}) \times \text{weight in kg}}{\text{serum creatinine in mg/dl} \times 72}$$

$$\text{for women:} \quad CLcr = \frac{(140 - \text{age in years}) \times \text{weight in kg}}{\text{serum creatinine in mg/dl} \times 85}$$

The 180-day mortality in patients with normal renal function and renal impairment is shown in Table 1.

Table 1. 180-day mortality in patients with normal or impaired renal function.

| Renal function | Mortality in levosimendan groups | Mortality in control groups |
|---|---|---|
| Normal<br>(CLcr > 80 ml/min) | 15 %<br>(24/156) | 24 %<br>(19/80) |
| Mild impairment<br>(CLcr 50 - 80 ml/min) | 18%<br>(38/206) | 35%<br>(24/68) |

The decrease in mortality was highest in patients with impaired renal function, which demonstrates the beneficial effects of levosimendan on impaired renal function.

References:

Lafayette RA, Perrone RD, Levey AS (1997). Laboratory evaluation of renal function in Diseases of the kidney. Edited by Schrier RW, Gottschalk CW, 6th edition, Vol 1, pages 314-319. Little, Brown and Company, Boston New York Toronto London.

## Claims

1. Use of levosimendan or its metabolite (II) (R)-N-[4-(1,4,5,6 - tetrahydro-4-methyl 6-oxo-3-pyridazinyl)phenyl]acetamide or any of their pharmaceutically acceptable salts in the manufacture of a medicament for the treatment of renal failure.

2. Use of levosimendan or its metabolite (II) or any of their pharmaceutically acceptable salts in the manufacture of a medicament for reducing mortality in a mammal suffering from renal failure the mortality being associated with the deterioration of kidney function.

## Patentansprüche

1. Verwendung von Levosimendan oder seines Metaboliten (II) (R)-N-[4-(1,4,5,6-Tetrahydro-4-methyl-6-oxo-3-pyridazinyl)phenyl]acetamid oder eines ihrer pharmazeutisch akzeptablen Salze zu Herstellung eines Medikamentes zur Behandlung von Niereninsuffizienz.

2. Verwendung von Levosimendan oder seines Metaboliten (II) oder eines ihrer pharmazeutisch akzeptablen Salze zu Herstellung eines Medikamentes zur Senkung der Mortalität bei einem Säuger, der an Niereninsuffizienz leidet, wobei die Mortalität mit der Verschlechterung der Nierenfunktion einhergeht.

## Revendications

1. Utilisation du levosimendan ou de son métabolite (II) acétamide de (R)-N-[4-(1,4,5,6-tetrahydro-4-méthyl-6-oxo-3-pyridazinyl)phényle] ou quelconque de ses sels pharmaceutiquement acceptables dans la fabrication d'un médicament pour le traitement de l'insuffisance rénale.

2. Utilisation du levosimendan ou de son métabolite (II) acétamide de (R)-N-[4-(1,4,5,6-tetrahydro-4-méthyl-6-oxopyridazinyl)phényle] ou quelconque de ses sels pharmaceutiquement acceptables dans la fabrication d'un médicament pour la réduction de la mortalité chez un mammifère atteint d'une insuffisance rénale, la mortalité étant associée à la détérioration de la fonction de reins.